# EUROPÄISCHE PATENTANMELDUNG

(11) **EP 1 024 128 A1**
(43) Veröffentlichungstag der Anmeldung: **02.08.2000**
(21) Anmeldenummer: 99126266.8
(22) Anmeldetag: 31.12.1999
(51) Int. Cl.: C07C 37/74

(54) **Verfahren zum Entfernen von Hydroxyaceton aus hydroxyacetonhaltigem Phenol**

(30) Priorität: 29.01.1999 DE 19903414
(71) Anmelder: Phenolchemie GmbH & Co. KG, 45966 Gladbeck (DE)
(72) Erfinder: van Barneveld, Heinrich, Dr., 46244 Bottrop (DE); Gerlich, Otto, Dr., 45966 Gladbeck (DE)

(57) **Zusammenfassung**

Bei den herkömmlichen Verfahren zur Entfernung von Hydroxyaceton aus hydroxyacetonhaltigem Phenol wurde ein relativ großer Energieeinsatz benötigt und/oder es ging Phenol verloren.

Die vorliegende Erfindung betrifft deshalb eine Verfahren zur Entfernung von Hydroxyaceton aus hydroxyacetonhaltigem Phenol, welches bei der säurekatalysierten Spaltung von Cumolhydroperoxid entsteht, durch Behandeln des dampfförmigen hydroxyacetonhaltigen Phenols mit einer Base. Bei dem erfindungsgemäßen Verfahren werden Destillations- und Reaktionsschritt in einer Weise kombiniert, daß entweder eine am Kopf einer Destillationskolonne abgenommene dampfförmige Phase in einen Reaktor gebracht wird, in welchem diese Phase als Dampf mit der Base reagiert, oder die Reaktion in einer Destillationskolonne stattfindet, in die nicht nur das zu destillierende hydroxyacetonhaltige Phenol, sondern auch die Base eingespeist wird.

Der Vorteil des erfindungsgemäßen Verfahrens gegenüber Verfahren, welche in bekannter Weise ausgeführt werden, liegt im geringeren Energieeinsatz, der zur Abtrennung des Hydroxyacetons benötigt wird. Zusätzlich kann der Phenolverlust, der bei anderen Verfahren zum Teil erheblich ist, verringert werden.

Das erfindungsgemäße Verfahren wird unter anderem bei der Aufbereitung von bei der Hock'schen Phenolsynthese anfallendem Rohphenol genutzt.

## Beschreibung

Die vorliegende Erfindung betrifft ein Verfahren zum Entfernen von Hydroxyaceton aus hydroxyacetonhaltigem Phenol.

Bei der säurekatalysierten Spaltung von Cumolhydroperoxid, welches nach dem Hock'schen Verfahren durch Oxidation von Cumol erhalten wurde, entsteht als ein unerwünschtes Nebenprodukt Hydroxyaceton. Die Konzentrationen des Hydroxyacetons liegen bei der heterogenen Spaltung von Cumolhydroperoxid bei ca. 400 bis 700 ppm und bei der homogenen Spaltung von Cumolhydroperoxid bei ca. 1 000 bis 2 000 ppm. Das Hydroxyaceton läßt sich, obwohl die beiden Verbindungen eine um 37 °C unterschiedliche Siedetemperatur aufweisen, durch einfaches Destillieren nicht vom Phenol abtrennen, weil zwischen Hydroxyaceton und Phenol polare Wechselwirkungen auftreten. Da das Hydroxyaceton unter Bedingungen, wie sie üblicherweise bei der Aufarbeitung von Rohphenol herrschen, mit diesem und/oder mit anderen Nebenprodukten wie z.B. α-Methylstyrol, Mesityloxid oder Acetophenon, die bei der Phenolsynthese nach dem Hock'schen Verfahren ebenfalls entstehen können, reagiert, muß das Hydroxyaceton vor der endgültigen Reinigung des Phenols abgetrennt werden.

In EP 0 505 146 wird ein Verfahren beschrieben, in dem Phenol Verunreinigungen, unter anderem Hydroxyaceton, durch Wasserextraktivdestillation entzogen werden.

In US 4 251 325 wird Phenol von Hydroxyaceton durch eine spezielle Ausführung einer fraktionierten Destillation befreit.

Aus BP 883 746 ist ein Verfahren bekannt, bei dem der mit Hydroxyaceton verunreinigten flüssigen Phenolphase eine Base zugesetzt wird. Durch die Base katalysiert, reagiert das Hydroxyaceton zu Produkten mit hinreichenden Siedepunktunterschieden, die sich durch eine einfache Destillation vom Phenol trennen lassen.

In BP 920 905 wird ein Verfahren beschrieben, in dem zur Reinigung des hydroxyacetonhaltigen Phenols der flüssigen Phenolphase erst Base und nach einer destillativen Aufbereitung Eisenchlorid zugesetzt werden.

Aus US 2 971 893 ist ein Verfahren bekannt, bei welchem die Reinigung des Phenols dadurch erreicht wird, daß der flüssigen Phenolphase eine basische Wasserstoffperoxid-Lösung zugegeben wird und anschließend destilliert wird.

US 4 298 765 lehrt ein Verfahren, bei dem in flüssiger Phase dem zu reinigenden Phenol ebenfalls eine Base zugesetzt wird. In einem zweiten Schritt wird diesem Gemisch eine Säure zugesetzt und anschließend wird eine Wasserdampfdestillation durchgeführt. Das im Sumpf verbliebene Phenol wird über Vakuumdestillation weiter gereinigt, und man erhält hochreines Phenol.

Aus US 4 634 796 ist ebenfalls ein Verfahren bekannt, bei welchem dem zu reinigenden Phenol Base zugesetzt wird. Hier wird das Gemisch direkt einer Wasserdampfdestillation unterzogen. Erst der Sumpf, der überwiegend Phenol enthält, wird mit Säure behandelt, und dieses Gemisch wird einer weiteren Destillation zugeführt, um das hochreine Phenol abzutrennen.

Alle diese vorgenannten Verfahren mit basischer Behandlung des Phenols benötigen zur Herstellung von hochreinem Phenol einen sehr hohen Energieeinsatz, da nach der Reaktion des zu behandelnden Phenols mit der Base in flüssiger Phase das Phenol immer durch eine Destillation abgetrennt werden muß.

In US 5 414 154 ist ein weiteres Verfahren beschrieben, welches unter Verwendung eines sauren Ionenaustauschers und/oder eines supersauren Katalysators zur Abtrennung von Methylbenzofuran aus Phenol eingesetzt werden kann. Bei diesem Verfahren wird das Methylbenzofuran durch Einbau eines weiteren Moleküls Phenol in eine Verbindung überführt, die sich leicht destillativ von Phenol trennen läßt. Dieses Verfahren läßt sich auch zur Entfernung von Hydroxyaceton aus Phenol einsetzen, wenn ebenfalls säurekatalysiert Hydroxyaceton mit Phenol zu Methylbenzofüran umgesetzt wird und dieses wiederum, durch Einbau eines weiteren Moleküls Phenol, in eine Verbindung überführt werden kann, die sich leicht destillativ von Phenol abtrennen läßt. Bei diesem Verfahren gehen jedoch aufgrund des Reaktionsmechanismus pro mol Hydroxyaceton als Verunreinigung zwei mol Phenol verloren.

Der Erfindung liegt daher die Aufgabe zugrunde, ein einfaches Verfahren zum Entfernen von Hydroxyaceton aus hydroxyacetonhaltigem Phenol bereitzustellen, welches einen möglichst geringen Energieeinsatz erfordert und gleichzeitig den Verlust an Phenol minimiert.

Es wurde nun überraschenderweise gefunden, daß das Entfernen von Hydroxyaceton aus hydroxyacetonhaltigem Phenol durch Zusatz von zumindest einer Base zu hydroxyacetonhaltigem Phenol in der Dampfphase wesentlich vereinfacht wird und der benötigte Energieeinsatz sowie die Phenolverluste deutlich verringert werden.

Gegenstand der vorliegenden Erfindung ist daher ein Verfahren zum Entfernen von Hydroxyaceton aus hydroxyacetonhaltigem Phenol, das dadurch gekennzeichnet ist, daß das hydroxyacetonhaltige Phenol in der Dampfphase mit zumindest einer Base behandelt wird.

Mit Hilfe des erfindungsgemäßen Verfahrens kann aus hydroxyacetonhaltigem Phenol überraschend einfach Hydroxyaceton entfernt werden. Ein wesentlicher Vorteil gegenüber den bekannten Verfahren besteht darin, daß durch Zugabe der Base zum hydroxyacetonhaltigen Phenol in der Dampfphase die thermische Energie des Phenols genutzt wird, um gleichzeitig eine Destillation mit deutlich geringerem Energieeinsatz - es muß nur soviel Energie zugeführt werden, daß die Wärmeverluste ausgeglichen werden - vornehmen zu können. Bei den bekannten Verfahren wird das hydroxyacetonhaltige Phenol, welches als Kopfprodukt aus der vorherigen destillativen Bearbeitungsstufe anfällt, erst kondensiert, dann mit einer Base versetzt und anschließend nochmals destilliert. Die Kondensationswärme, die bei der Kondensation des Phenols anfällt, kann in dem erfindungsgemäßen Verfahren ebenso wie bei den bekannten Verfahren genutzt werden, mit dem Unterschied, daß das Phenol erfindungsgemäß erst nach Verlassen der Reaktions- bzw. Destillationskolonne, die der Entfernung des Hydroxyacetons dient, - also nur einmal - kondensiert wird. Durch Einsatz des erfindungsgemäßen Verfahrens wird außerdem verhindert, daß Phenol durch die Umsetzung mit Hydroxyaceton verloren geht. Das erfindungsgemäße Verfahren ermöglicht also in bezug auf Ausbeute und Energieeinsatz eine wesentlich wirtschaftlichere Produktion von Phenol.

Das erfindungsgemäße Verfahren wird im folgenden beispielhaft anhand der Reinigung von Rohphenol, welches nach der Hock'schen Synthese hergestellt wird, näher beschrieben.

Bei dieser in bekannter Weise durchgeführten Synthese wird aus Cumol und Sauerstoff Cumolhydroperoxid hergestellt. Dieses wird in einer zum Beispiel in homogener Phase durchgeführten Spaltreaktion säurekatalysiert in Phenol und Aceton aufgespalten. In dem gesamten Prozeß entstehen nicht nur Aceton und Phenol, sondern das Spaltproduktgemisch kann zusätzlich noch Cumol, Methylbenzofüran, Hydroxyaceton, α-Methylstyrol sowie weitere Nebenprodukte enthalten.

Die Abtrennung von Aceton und Cumol aus dem Spaltproduktgemisch geschieht üblicherweise durch Destillation. In einer ersten Destillationskolonne wird das Aceton abgetrennt. Das im Sumpf anfallende Rohphenol wird im allgemeinen zum Abtrennen des Cumols in eine weitere Destillationskolonne gefahren. In dieser wird das Cumol über Kopf abgenommen. Das Rohphenol, welches im Sumpf abgezogen wird, wird einer weiteren destillativen Reinigung unterzogen.

In dieser nachfolgenden Reinigungsstufe wird das Rohphenol üblicherweise in eine Destillationskolonne gefahren und über Kopf abgezogen. Höhersiedende Verunreinigungen, wie z.B. polymerisiertes α-Methylstyrol, bleiben im Sumpf zurück und können einer Aufarbeitung, z.B. dem Cracken zur Rückgewinnung von Phenol und/oder Cumol, zugeführt werden.

In herkömmlichen Verfahren erfolgt eine Kondensation des Rohphenols unter Nutzung der Kondensationswärme und eine weitere destillative Aufarbeitung.

Erfindungsgemäß wird das am Kopf dampfförmig abgenommene Gemisch, welches außer hydroxyacetonhaltigem Phenol auch noch Verbindungen enthält, die einen niedrigeren Siedepunkt als Phenol aufweisen, nun dem erfindungsgemäßen Verfahren zugeführt. Zu diesem Zweck wird das Gemisch dampfförmig, also ohne Kondensation, in diesem Beispiel in eine Reaktionskolonne gefahren. Oberhalb des Gemisches wird erfindungsgemäß zumindest eine Base, vorzugsweise Phenolatlauge, eine beliebige organische Base oder Alkalihydroxid-Lösung, so in die Reaktionskolonne gefahren, daß sie dem dampfförmigen Gemisch entgegenströmt.

Durch die dem dampfförmigen, hydroxyacetonhaltiges Phenol und Leichtsieder enthaltenden Gemisch entgegenströmende Base wird überraschenderweise eine basisch katalysierte Kondensationsreaktion des Hydroxyacetons erreicht. Das Produkt aus dieser Kondensationsreaktion, welches harzartig ist und in der Base gelöst vorliegt, hat einen höheren Siedepunkt als das Phenol und reichert sich dadurch im Sumpf der Kolonne an.

Von der Base, die sich ebenfalls im Sumpf der Reaktionskolonne sammelt und die zum Teil die Kondensationsprodukte des Hydroxyacetons enthält, wird kontinuierlich oder diskontinuierlich, vorzugsweise kontinuierlich, soviel abgezogen, daß das Flüssigkeitsvolumen, welches sich im Sumpf der Reaktionskolonne befindet, annähernd konstant bleibt. Die abgezogene Base wird ganz oder teilweise vorzugsweise wieder oberhalb der Gemischeinspeisung in die Kolonne eingespeist oder ganz oder teilweise einer Aufarbeitung oder Entsorgung, im Fall von Phenolatlauge als Base vorzugsweise der Phenolatlaugezerlegung, welche in bekannter Weise durch Säure erfolgt, zugeführt.

Zum Erhalt der Reaktivität der Base in der Reaktionskolonne kann erfindungsgemäß kontinuierlich oder diskontinuierlich, vorzugsweise kontinuierlich, soviel Base, vorzugsweise Phenolatlauge, eine organische Base oder Alkalihydroxid-Lösung, in die Reaktionskolonne oberhalb oder unterhalb, vorzugsweise oberhalb, der Gemischeinspeisung, gefahren werden, daß die durch die Reaktion verbrauchte Menge an Base ersetzt wird.

Damit das von Hydroxyaceton gereinigte Phenol und Leichtsieder enthaltende Gemisch am Kopf der Reaktionskolonne abgezogen werden kann, muß die Reaktionskolonne zum Ausgleich von Wärmeverlusten der dampfförmigen Einträge geringfügig zusatzbeheitzt werden, damit das Gemisch während der gesamten Verweilzeit in der Reaktionszone dampfförmig vorliegt. Die Temperatur in der Reaktionskolonne beträgt erfindungsgemäß zwischen 100 und 300 °C, bevorzugt zwischen 150 und 250 °C und besonders bevorzugt zwischen 170 und 190 °C. Die Beheizung der Reaktionskolonne kann in bekannter Weise, z.B. mit Wärmeträgern oder mit überhitztem Dampf, erfolgen.

Das am Kopf der Reaktionskolonne abgezogene von Hydroxyaceton befreite Gemisch kann unter Nutzung der Kondensationswärme kondensiert und einer weiteren Aufarbeitung, z.B. der destillativen Abtrennung leichtersiedender Verbindungen vom Phenol, zugeführt werden.

Als Reaktionskolonne kann eine üblicherweise für Reaktionen zwischen Gasen und Flüssigkeiten eingesetzte Apparatur, die eine möglichst große Kontaktoberfläche für die Reaktion zwischen Gasphase und flüssiger Phase zur Verfügung stellt, wie z.B. eine Destillationskolonne, verwendet werden, deren Größe so ausgelegt werden muß, daß kein Alkali, welches z.B. durch den Einsatz von Alkalihydroxid oder Phenolatlauge als Base in die Reaktionskolonne eingebracht wird, mit dem von Hydroxyaceton gereinigtem Gemisch die Reaktionskolonne verläßt, da dieses bei weiteren Aufarbeitungsschritten z. B. bei der Verwendung eines Ionenaustauschers dessen Effektivität stören würde. Die Dimensionierung der Kolonne kann durch einfache Vorversuche ermittelt werden.

In einer besonderen Ausführungsart des erfindungsgemäßen Verfahrens kann auf die Reaktionskolonne in der Weise verzichtet werden, daß als Reaktionskolonne eine schon vorhandene Destillationskolonne verwendet wird, in welche eine Reaktionszone integriert wird.

Ausgangsprodukt ist wiederum das hydroxyacetonhaltige Phenol, welches bereits von Hochsiedern befreit wurde und welches noch Verunreinigungen enthält, die einen niedrigeren Siedepunkt als Phenol haben. Sollen diese nicht aufeinander folgend, sondern in einem einzigen Verfahrensschritt gemeinsam mit dem Hydroxyaceton aus dem Rohphenol entfernt werden, so wird dieses Gemisch unterhalb des Kopfes, vorzugsweise zwischen erstem und fünftem Boden oberhalb des Sumpfes flüssig oder dampfförmig in die Destillationskolonne gefahren. Die Temperatur in der Destillationskolonne an der Gemischeinspeisung muß so gewählt werden, daß eine Reaktionszone verwirklicht wird, in welcher das überwiegend dampfförmige Gemisch mit der Base reagieren kann.

Zumindest eine Base, vorzugsweise Phenolatlauge, eine organische Base oder Alkalihydroxid-Lösung wird bei dieser besonderen Ausführungsart des erfindungsgemäßen Verfahrens oberhalb der Gemischeinspeisung in die Kolonne eingespeist, so daß die Base dem hydroxyacetonhaltigen, dampfförmigen Gemisch entgegenströmt. Der Abstand von Gemischeinspeisung zu Baseneinspeisung ist so groß zu wählen, daß eine genügend große Anzahl theoretischer Böden zwischen ihnen liegt, so daß eine Reaktionszone verwirklicht wird.

Das von Hydroxyaceton gereinigte Phenol kann seitlich oberhalb der Baseneinspeisung flüssig aus der Kolonne abgezogen werden. Der Abstand an theoretischen Böden zwischen Baseneinspeisung und Entnahmestelle für das gereinigte Phenol ist so zu wählen, daß ein Austrag an Alkali, z.B. in Form von Phenolatlauge, mit dem abgezogenen Phenol sicher verhindert wird.

Am Kopf der Kolonne wird ein Gemisch dampfförmig abgenommen, welches hauptsächlich Verbindungen enthält, die bei Temperaturen unterhalb der Siedetemperatur von Phenol sieden. Ein Teil dieses Gemisches kann kondensiert als Rücklauf in den oberen Teil der Kolonne zurückgefahren werden. Es können auch andere geeignete Mittel, wie z.B. Wasser, alleine oder als Zusatz zum am Kopf der Kolonne abgenommenen kondensierten Gemisch als Rücklauf in den oberen Teil der Kolonne gefahren werden.

Die durch basisch katalysierte Kondensation aus dem Hydroxyaceton entstandenen harzartigen Kondensationsprodukte, welche in der Base gelöst vorliegen, werden im Sumpf der Kolonne angereichert. Aus dem Sumpf der Kolonne können die Kondensationsprodukte zusammen mit der Base, die sich ebenfalls im Sumpf der Kolonne sammelt, kontinuierlich oder diskontinuierlich, vorzugsweise kontinuierlich, abgezogen werden, so daß das Flüssigkeitsvolumen im Sumpf der Destillationskolonne annähernd konstant bleibt. Ein Teil der abgezogenen, die Kondensationsprodukte des Hydroxyacetons enthaltenden Base kann wieder oberhalb der Phenoleinspeisung in die Destillationskolonne eingespeist werden. Ein anderer Teil der abgezogenen Base kann der Entsorgung oder Aufarbeitung, im Fall von Phenolatlauge als eingesetzte Base der Phenolatlaugezerlegung, zugeführt werden.

Zum Erhalt der Reaktivität der Base in der Destillationskolonne kann erfindungsgemäß kontinuierlich oder diskontinuierlich, vorzugsweise kontinuierlich, soviel Base, vorzugsweise Phenolatlauge, eine organische Base oder Alkalihydroxid-Lösung, in die Destillationskolonne oberhalb der Gemischeinspeisung gefahren werden, daß die durch die Reaktion verbrauchte Menge an Base ersetzt wird.

Die Temperatur in der Destillationskolonne ist so zu wählen, daß das hydroxyacetonhaltige Gemisch in der Reaktionszone, in der es mit der Base reagieren soll, überwiegend dampfförmig vorliegt. Die Temperatur in der Destillationskolonne beträgt erfindungsgemäß zwischen 100 und 300 °C, bevorzugt zwischen 150 und 250 °C und besonders bevorzugt zwischen 170 und 190 °C. Die Beheizung der Destillationskolonne kann in bekannter Weise z.B. mit Wärmeträgern oder mit überhitztem Dampf, erfolgen.

Als Destillationskolonne kann eine üblicherweise für Destillationen eingesetzte Kolonne verwendet werden, deren Größe so ausgelegt werden muß, daß kein Alkali mit dem von Hydroxyaceton gereinigtem Phenol die Destillationskolonne verläßt, da dieses bei weiteren Aufarbeitungsschritten z. B. bei der Verwendung eines Ionenaustauschers dessen Effektivität stören würde. Die Dimensionierung der Kolonne sowie der Abstand an theoretischen Böden zwischen den Einspeisestellen und den Entnahmestellen kann durch einfache Vorversuche ermittelt werden.

Bei der oben beschriebenen besonderen Ausführungsart des erfindungsgemäßen Verfahrens kann, im Vergleich zur als erstes beschriebenen Ausführungsart des erfindungsgemäßen Verfahrens, auf eine gesonderte Reaktionskolonne verzichtet werden.

Eine weitere besondere Ausführungsart des erfindungsgemäßen Verfahrens, die sich einer Kombination von Destillations- und Reaktionskolonne bedient, kann angewendet werden, wenn in einem Schritt das Phenol sowohl von Hydroxyaceton als auch von höher und niedriger siedenden Verbindungen abgetrennt werden soll.

Das Rohphenol wird bei diesem Verfahren unterhalb des Kopfes, vorzugsweise unmittelbar oberhalb des Sumpfes, in die Destillationskolonne gefahren. Durch das Temperaturprofil in der Kolonne wird das Rohphenol in der Weise aufgetrennt, daß sich Verbindungen mit höheren Siedetemperaturen als Phenol im Sumpf der Kolonne anreichern, Verbindungen die eine niedrigere Siedetemperatur als Phenol überwiegend im Kopf der Destillationskolonne anreichern und sich gereinigtes Phenol seitlich flüssig entnehmen läßt. Das gereinigte Phenol wird seitlich, etwa bei einem Verhältnis der theoretischen Böden der Kolonne oberhalb zu unterhalb der Entnahmestelle von 1 zu 3, bevorzugt von 1 zu 3,5 und besonders bevorzugt von 1 zu 4, aus der Destillationskolonne abgezogen. Aus dem Sumpf der Destillationskolonne, in dem sich ein Gemisch, welches überwiegend die Verunreinigungen enthält, die eine höhere Siedetemperatur als Phenol haben, angereichert hat, kann dieses Gemisch kontinuierlich oder diskontinuierlich, vorzugsweise kontinuierlich, abgezogen werden. Die abgezogenen Verunreinigungen können einer Aufarbeitung zugeführt werden. Am Kopf der Destillationskolonne wird ein Gemisch entnommen, welches überwiegend die leichtersiedenden Verunreinigungen sowie Hydroxyaceton und Phenol enthält.

Dieses dampfförmige Gemisch wird erfindungsgemäß direkt in eine Reaktionskolonne gefahren. Oberhalb des Gemisches wird eine Base, vorzugsweise Phenolatlauge, eine beliebige organische Base oder Alkalihydroxid, besonders bevorzugt Phenolatlauge, in die Reaktionskolonne gefahren, so daß die Base dem zu reinigenden dampfförmigen Gemisch entgegenströmt.

Durch die Base wird die Kondensationsreaktion des Hydroxyaceton katalysiert und es entsteht ein höhersiedendes harzartiges Kondensationsprodukt, welches sich im Sumpf sammelt und in der Base gelöst vorliegt. Ein Teil des am Kopf der Reaktionskolonne abgezogenen an Hydroxyaceton abgereicherten Dampfes kann kondensiert oben in die Destillationskolonne zurückgefahren werden. Ein weiterer Teil kann bei Bedarf wegen des Gehalts an Leichtsiedern anderen Aufbereitungsschütten, z.B. früheren Destillationsachritten im Gesamtprozeß der Phenolherstellung, zugeführt werden. Ein gegebenenfalls dritter Teil des am Kopf der Reaktionskolonne kondensierten Dampfes kann in die Reaktionskolonne zurückgefahren werden. Es kann vorteilhaft sein, die Teilströme an kondensiertem Kopfprodukt durch auf dem Fachmann bekannte konstruktive Maßnahmen mengenmäßig regulieren zu können, so daß je nach Zusammensetzung der Teilströme diese so eingestellt werden können, daß entweder mehr kondensierter Dampf in den Kopf der Reaktionskolonne, in den Kopf der Destillationskolonne oder der Aufarbeitung zugeführt werden kann. Durch konstruktive Maßnahmen kann das Verfahren so ausgeführt werden, daß die Teilströme von 0 bis 100 % des gesamten, vorzugsweise kondensierten, Dampfes aufnehmen können.

Aus dem Sumpf der Reaktionskolonne, in welchem sich das Kondensationsprodukt des Hydroxyacetons sowie die Base sammelt, kann kontinuierlich oder diskontinuierlich, vorzugsweise kontinuierlich, soviel Kondensationsprodukt enthaltende Base abgezogen werden, daß der Flüssigkeitsstand in der Reaktionskolonne annähernd konstant bleibt. Die abgezogene Base kann, abhängig vom Grad der Verunreinigung und von der verbleibenden Reaktivität, der Aufarbeitung, im Fall von Phenolatlauge als Base vorzugsweise der Phenolatlaugezerlegung, zugeführt oder oberhalb der Gemischeinspeisung, in der das am Kopf der Destillationskolonne abgenommene Gemisch dampfförmig eingespeist wird, wieder in die Reaktionskolonne eingespeist werden.

Zur Aufrechterhaltung der Reaktivität der Base in der Reaktionskolonne kann erfindungsgemäß kontinuierlich oder diskontinuierlich, vorzugsweise kontinuierlich soviel Base, vorzugsweise Phenolatlauge, eine beliebige organische Base oder Alkalihydroxid-Lösung, in die Reaktionskolonne oberhalb oder unterhalb, vorzugsweise oberhalb, der Gemischeinspeisung, gefahren werden, daß die durch die Reaktion verbrauchte Menge an Base ersetzt wird.

Als Destillationskolonne kann eine üblicherweise für Destillationen eingesetzte Kolonne verwendet werden, deren Größe so gewählt werden muß, daß die erfindungsgemäße Abtrennung des Phenols von leichter- und höhersiedenden Verbindungen erreicht wird. Als Reaktionskolonne kann eine üblicherweise für Reaktionen zwischen Gasen und Flüssigkeiten eingesetzte Apparatur, wie z.B. eine Destillationskolonne, verwendet werden, deren Größe so ausgelegt werden muß, daß kein Alkali mit dem von Hydroxyaceton gereinigtem Phenol die Reaktionskolonne verläßt, da dieses bei weiteren Aufarbeitungsschritten z. B. bei der Verwendung eines Ionenaustauschers dessen Effektivität stören würde. Die Größe der Kolonnen, sowie die Abstände an theoretischen Böden zwischen den Einspeisestellen und den Entnahmestellen können durch Vorversuche ermittelt werden.

Damit das von Hydroxyaceton gereinigte Phenol und Leichtsieder enthaltende Gemisch am Kopf der Reaktionskolonne abgezogen werden kann, muß die Reaktionskolonne zum Ausgleich von Wärmeverlusten der dampfförmigen Einträge geringfügig zusatzbeheitzt werden, damit das Gemisch während der gesamten Verweilzeit in der Reaktionszone dampfförmig vorliegt.

Die Temperatur in der Reaktionskolonne sowie in der Destillationskolonne beträgt erfindungsgemäß zwischen 100 und 300 °C, bevorzugt zwischen 150 und 250 °C und besonders bevorzugt zwischen 170 und 190 °C. Die Beheizung der Reaktionskolonne kann in bekannter Weise, z.B. mit Wärmeträgern oder mit überhitztem Dampf erfolgen.

Um bei der Durchführung des erfindungsgemäßen Verfahrens die Bildung eines Feststoffes, welcher durch weitere Kondensationsreaktionen der Kondensationsprodukte des Hydroxyacetons mit sich selbst oder mit Hydroxyaceton entstehen kann, zu vermeiden, kann es vorteilhaft sein, einen Teil des sich im Sumpf der Reaktions- oder Destillationskolonne sammelnden und in der Base gelöst vorliegenden Kondensationsproduktes kontinuierlich oder diskontinuierlich zusammen mit der Base aus dem Prozess auszuschleusen. Die durchschnittliche maximale Verweildauer der Kondensationsprodukte im Prozeß, bis diese zu Feststoffen kondensieren und nicht mehr in der Base löslich sind, kann durch Vorversuche bestimmt werden.

Ebenso kann es vorteihaft sein, die Kondensationsprodukte solange im Prozeß zu belassen, bis diese durch weitere Kondensationsreaktionen mit sich selbst oder mit dem Hydroxyaceton Feststoffe bilden, die sich nicht mehr in der Base lösen. Diese Feststoffe können durch zumindest eine Apparatur, die zur Abtrennung von Feststoffen aus flüssigen Phasen geeignet ist, z.B. einen Filter, weiche in eine Leitung, die die aus dem Sumpf abgezogene Base führt, eingebaut werden kann, abgetrennt und somit aus dem Prozeß entfernt werden. Die abgetrennten Feststoffe können einer Aufarbeitung, z.B. der thermischen Verwertung, zugeführt werden.

Das erfindungsgemäße Verfahren kann unter Umgebungsdruck, erhöhtem Druck oder erniedrigtem Druck, vorzugsweise unter Umgebungsdruck, ausgeführt werden.

Zur Steigerung der Abtrennleistung bei dem Entfernen von Hydroxyaceton aus hydroxyacetonhaltigem Phenol oder weiteren im Phenol vorhandenen Verunreinigungen kann es zweckmäßig sein, eine oder mehrere der Ausführungsarten des erfindungsgemäßen Verfahrens zu kombinieren oder hintereinander zu schalten.

Zur Regulierung der in die Kolonnen ein- und austretenden Stoffströme sowie zur Regulierung von Teilströmen kann es vorteihaft sein, dem Fachmann bekannte Regulierungsmöglichkeiten, wie z.B. Ventile oder Hähne, in den die Stoff- oder Teilströme führenden Leitungen vorzusehen.

Das erfindungsgemäße Verfahren kann kontinuierlich oder diskontinuierlich ausgeführt werden.

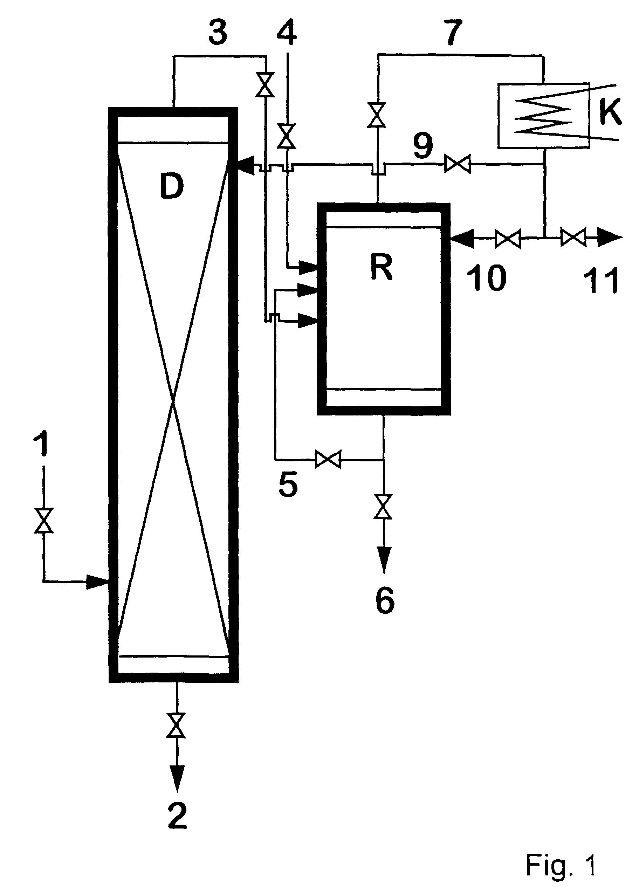

In Figur 1 ist beispielhaft eine Ausführungsart des erfindungsgemäßen Verfahrens dargestellt, ohne daß das Verfahren auf diese beschränkt ist.

Das zu behandelnde Phenol **1** wird in die Destillationskolonne D gefahren. Aus dem Sumpf der Destillationskolonne wird der Rückstand **2**, der Verbindungen enthält, die einen höheren Siedepunkt als Phenol haben, abgezogen. Am Kopf der Destillationskolonne wird das dampfförmige, hydroxyacetonhaltige Phenol **3** abgenommen und in die Reaktionskolonne R eingespeist. Aus dem Sumpf der Reaktionskolonne wird die Base, die das Kondensationsprodukt des Hydroxyacetons enthält entweder der Aufarbeitung, z.B. der Phenolatlaugezerlegung, zugeführt (Leitung **6**) oder wieder in die Reaktionskolonne oberhalb der Phenoleinspeisung eingespeist (Leitung **5**). Zum Ausgleich der über Leitung **6** abgezogenen Base wird der Reaktionskolonne oberhalb der Phenoleinspeisung frische Base **4** zugeführt. Am Kopf der Reaktionskolonne wird das an Hydroxyaceton abgereicherte Phenol abgenommen (Leitung **7**), welches unter Nutzung der Kondensationswärme kondensiert werden kann. Dazu wird das Kopfprodukt über einen Wärmetauscher **K** geleitet und kondensiert. Das kondensierte Gemisch wird entweder wieder zurück in die Destillationskolonne geleitet (Leitung **9**), in die Reaktionskolonne geleitet (Leitung **10**) oder einer Aufarbeitung, z.B. einer weiteren Destillation, zugeführt (Leitung **11**).

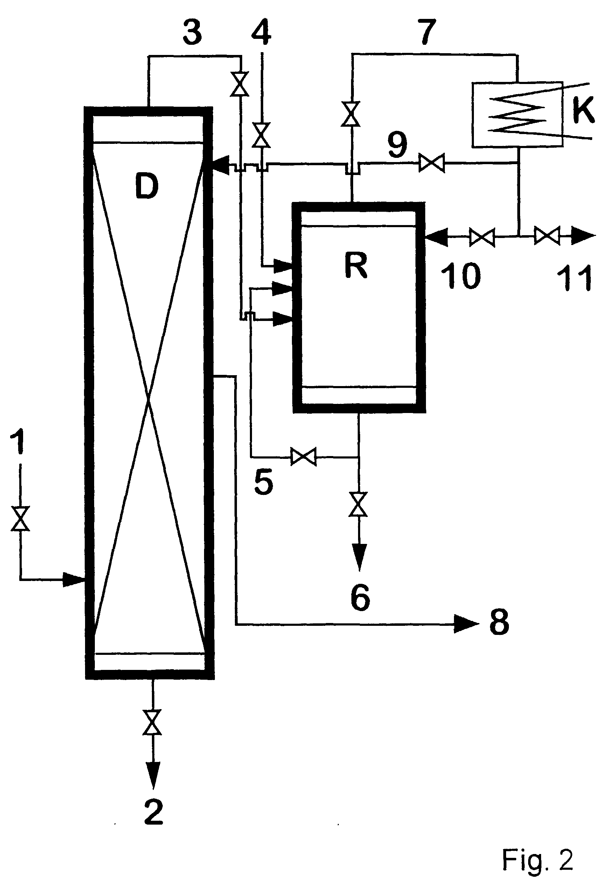

In Figur 2 ist beispielhaft eine weitere Ausführungsart des erfindungsgemäßen Verfahrens dargestellt, ohne daß das Verfahren auf diese beschränkt ist.

Das zu behandelnde Phenol **1** wird in die Destillationskolonne **D** gefahren. Aus dem Sumpf der Destillationskolonne wird der Rückstand **2**, der Verbindungen enthält, die einen höheren Siedepunkt als Phenol haben, abgezogen. Am Kopf der Destillationskolonne wird ein dampfförmiges Gemisch, welches Hydroxyaceton, leichtersiedende Verunreinigungen und geringe Mengen Phenol enthält, abgenommen und in die Reaktionskolonne **R** eingespeist (Leitung **3**). Aus dem Sumpf der Reaktionskolonne wird die Base, die das Kondensationsprodukt des Hydroxyacetons enthält, entweder der Aufarbeitung, z.B. der Phenolatlaugezerlegung zugeführt (Leitung **6**) oder wieder in die Reaktionskolonne oberhalb der Phenoleinspeisung eingespeist (Leitung **5**). Zum Ausgleich der über Leitung **6** abgezogenen Base wird der Reaktionskolonne oberhalb der Phenoleinspeisung frische Base **4** zugeführt. Am Kopf der Reaktionskolonne wird das an Hydroxyaceton abgereicherte Gemisch aus Leichtsiedern und geringen Mengen Phenol abgenommen (Leitung **7**). Dieses wird über einen Wärmetauscher **K** geleitet und kondensiert. Das kondensierte Gemisch wird entweder wieder zurück in die Destillationskolonne geleitet (Leitung **9**), in die Reaktionskolonne geleitet (Leitung **10**) oder einer Aufarbeitung, z.B. einer weiteren Destillation, zugeführt (Leitung **11**). Das von Hydroxyaceton weitgehend befreite Phenol **8** wird seitlich aus der Destillationskolonne abgezogen und weiteren Aufarbeitungs- oder Verarbeitungsschritten zugeführt.

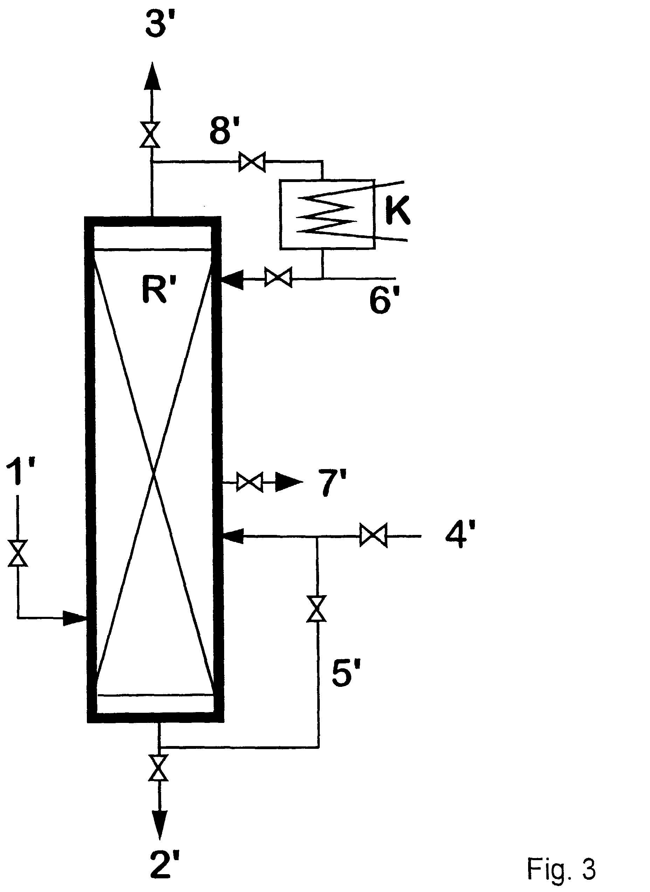

In Figur 3 ist beispielhaft eine weitere Ausführungsart des erfindungsgemäßen Verfahrens dargestellt, ohne daß das Verfahren auf diese beschränkt ist.

Das von Hochsiedern in einer Vorkolonne befreite Phenol wird über **1'** in den unteren Teil einer Reaktionskolonne **R'** eingespeist. Die im Phenol enthaltenen störenden Verbindungen wie Hydroxyaceton reagieren mit dem über **5'** im Kreislauf gefahrenen basischen Sumpfprodukt. Ein Teilstrom wird über **2'** abgezogen und der Verlust an Base über **4'** ergänzt. Über **3'** verlassen Leichtsieder die Kolonne und über **6'** wird ein geeignetes Mittel, wie z.B. Wasser als Rücklauf gefahren oder über Leitung **8'** wird ein Teil der Leichtsieder als Rücklauf gefahren. In der Leitung **8'** kann ein Wärmetauscher **K** vorgesehen sein, der es ermöglicht die Leichtsieder zu kondensieren. An **7'** wird das von Hydroxyaceton und Leichtsiedern weitestgehend befreite Phenol abgezogen und der Weiterverarbeitung zugeführt.

Das erfindungsgemäße Verfahren wird durch die folgenden Beispiele naher erläutert, ohne darauf beschränkt zu sein.

### Beispiel 1:

In einer Destillationskolonne wurden zu 80 g Phenol 8 g 50 %ige Natronlauge gegeben. Über Kurzweg wurde das durch die Natronlauge eingebrachte Wasser abdestilliert. Nach Entfernen des Wassers wurden 400 g/h Rohphenol mit einem Hydroxyacetongehalt von 1 000 bis 1 200 ppm über einen Zeitraum von 5 h zugetropft. Gleichzeitig wurde über den Kopf der Destillationskolonne 400 g/h Kopfprodukt abdestilliert. Das am Kopf der Kolonne abdestillierte Phenol hatte einen Hydroxyacetongehalt von 30 bis 65 ppm.

Das Beispiel zeigt, daß durch Zugabe von Natronlauge zum hydroxyacetonhaltigen Phenol das Hydroxyaceton weitgehend aus dem Phenol entfernt werden kann, da es ein Kondensationsprodukt bildet, welches sich im Sumpf der Kolonne anreichert

### Beispiel 2:

In eine Destillationskolonne wurden zu 40 g Phenol 8 g 50 %ige Natronlauge gegeben. Über einen Zeitraum von 8 h wurde 200 g/h Rohphenol mit 2,5 % Hydroxyaceton zugetropft. Über Kurzweg wurde die gleiche Menge Phenol abdestilliert. Nun wurde über einen Zeitraum von 4 h 250 g/h Phenol, welches 200 ppm 50 %iger Natronlauge und 1 000 bis 2 000 ppm Hydroxyaceton enthielt, zugetropft. Über Kopf wurden im gleichen Zeitraum 250 g/h an Kopfprodukt abdestilliert. Das Kopfprodukt enthielt 60 ppm Hydroxyaceton.

Nach Beendigung der Destillation wurde die Sumpf der Kolonne verbliebene Lösung mit 10 %iger Schwefelsäure neutralisiert. Dieses Gemisch wurde unter Zutropfen von 250 g/h hydroxyacetonfreiem Reinphenol über einen Zeitraum von 4 h destilliert.

Über Kopf wurden gleichzeitig 250 g/h Phenol abdestilliert. Der Hydroxyacetongehalt im Kopfprodukt betrug 70 ppm.

Das Beispiel zeigt, daß das im Sumpf der Destillationskolonne verbleibende Kondensationsprodukt so stabil ist, daß es nach Neutralisation des im Kolonnensumpf befindlichen Gemisches mit Schwefelsäure und unter anschließender Wärmeeinwirkung kaum Hydroxyaceton wieder freigibt.

### Beispiel 3

In einer Destillationskolonne wurde zu 50 g Phenol 1 g 50 %ige Natronlauge gegeben. Über einen Zeitraum von 4 h wurden 500 g Phenol, welches 1 % Hydroxyaceton enthält zugetropft. Es wurde gleichzeitig soviel Phenol über Kopf abdestilliert, daß die Sumpfmenge während der Dauer der Destillation immer 50 g betrug. Der Gehalt an Hydroxyaceton im Kopfprodukt betrug 100 ppm.

Dieser Versuch wurde wiederholt. Allerdings wurde oberhalb der Einspeisung des hydroxyacetonhaltigen Phenols 50 g des Sumpfgemisches aus dem vorangegangenem Versuch eingespeist. Das Kopfprodukt wies nun einen Hydroxyacetongehalt von 40 ppm auf.

An diesem Beispiel zeigt sich sehr deutlich, daß das Eingeben des Sumpfgemisches, welches das Kondensationsprodukt von Hydroxyaceton sowie Natronlauge enthält, oberhalb der Einspeisung des hydroxyacetonhaltigen Phenols, sich als vorteilhaft hinsichtlich der Effektivität der Abtrennung von Hydroxyaceton aus hydroxyacetonhaltigem Phenol erweist.

## Patentansprüche

1. Verfahren zum Entfernen von Hydroxyaceton aus hydroxyacetonhaltigem Phenol,
dadurch gekennzeichnet,
daß das hydroxyacetonhaltige Phenol in der Dampfphase mit zumindest einer Base behandelt wird.

2. Verfahren nach Anspruch 1,
dadurch gekennzeichnet,
daß das hydroxyacetonhaltige Phenol in einer Destillationskolonne mit einer Base behandelt wird.

3. Verfahren nach Anspruch 1 oder 2,
dadurch gekennzeichnet,
daß das Hydroxyaceton basisch katalysiert in Verbindungen umgewandelt wird, welche einen höheren Siedepunkt als Phenol aufweisen.

4. Verfahren nach zumindest einem der Ansprüche 1 bis 3,
dadurch gekennzeichnet,
daß als Base Alkalihydroxid eingesetzt wird.

5. Verfahren nach zumindest einem der Ansprüche 1 bis 3,
dadurch gekennzeichnet,
daß als Base eine organische Base eingesetzt wird.

6. Verfahren nach zumindest einem der Ansprüche 1 bis 3,
dadurch gekennzeichnet,
daß als Base Phenolatlauge eingesetzt wird.

7. Verfahren nach zumindest einem der Ansprüche 1 bis 6,
dadurch gekennzeichnet,
daß die im Sumpf anfallende Base oberhalb der Einspeisung des hydroxyacetonhaltigen Phenols wieder in die Destillations- oder Reaktionskolonne eingespeist wird.

8. Verfahren nach zumindest einem der Ansprüche 1 bis 7,
dadurch gekennzeichnet,
daß die Temperatur zwischen 100 und 300 °C liegt.

9. Verfahren nach Anspruch 8,
dadurch gekennzeichnet,
daß die Temperatur zwischen 150 und 250 °C liegt.

10. Verfahren nach Anspruch 9,
dadurch gekennzeichnet,
daß die Temperatur zwischen 170 und 190 °C liegt.
